# EUROPEAN PATENT APPLICATION

(11) **EP 2 578 258 A1**
(43) Date of publication of application: **10.04.2013**
(21) Application number: 11184101.1
(22) Date of filing: 06.10.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (2) comprising a needle hub (2.2), a needle (2.3) coupled to the needle hub (2.2) and having a distal tip (2.3.1); a needle shield (2.1) telescopically coupled to the needle hub (2.2), the needle shield (2.1), a locking element (2.4) including latch arms (2.4.2) adapted to engage the needle hub (2.2) and engagement elements (2.4.3) adapted to engage the needle shield (2.1). When the needle shield (2.1) is in a first axial position (PA1) relative to the needle hub (2.2), the needle shield (2.1) covers the distal tip (2.3.1) of the needle (2.3) and the engagement elements (2.4.3) of the locking element (2.4) engage the needle shield (2.1). When the needle shield (2.1) is in a second axial position (PA2) relative to the needle hub (2.2), the needle shield (2.1) is retracted to expose the distal tip (2.3.1) of the needle (2.3) and the latch arms (2.4.2) engage the needle hub (2.2). When the needle shield (2.1) is in a third axial position (PA3) relative to the needle hub (2.2), the needle shield (2.1) covers the distal tip (2.3.1) of the needle (2.3) and the engagement elements (2.4.3) abut the needle shield (2.1) to prevent proximal movement of the needle shield (2.1) relative to the needle hub (2.2).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a needle safety device according to the present invention comprises a needle hub, a needle coupled to the needle hub and having a distal tip; a needle shield telescopically coupled to the needle hub, the needle shield, a locking element including latch arms adapted to engage the needle hub and engagement elements adapted to engage the needle shield. When the needle shield is in a first axial position relative to the needle hub, the needle shield covers the distal tip of the needle and the engagement elements of the locking element engage the needle shield. When the needle shield is in a second axial position relative to the needle hub, the needle shield is retracted to expose the distal tip of the needle and the latch arms engage the needle hub. When the needle shield is in a third axial position relative to the needle hub, the needle shield covers the distal tip of the needle and the engagement elements abut the needle shield to prevent proximal movement of the needle shield relative to the needle hub.

In an exemplary embodiment, the needle hub includes a stem having a recess adapted to receive the latch arms of the locking element. The needle shield is moved from the first axial position to the second axial position, the latch arms are deflected by the stem. When the needle shield reaches the second axial position, the latch arms return to a non-deflected position to engage the recess. The latch arms include fingers adapted to engage the recess.

In an exemplary embodiment, the needle hub includes axial arms having radial protrusions adapted to engage axial slots on the needle shield.

In an exemplary embodiment, the needle shield includes axial ribs formed an inner surface. The ribs include a distal end adapted to engage the engagement elements in the first axial position and a proximal end adapted to abut the engagement elements in the third axial position. The distal end includes a ramped surface adapted to engage a corresponding ramped surface of the engagement elements. When the needle shield is moved from the first axial position to the second axial position, the engagement elements are deflected by the ribs. When the needle shield reaches the third axial position, the engagement elements return to a non-deflected position to engage the proximal end of the ribs.

In an exemplary embodiment, the needle safety device further comprises a spring disposed between the needle hub and the needle shield, the spring biasing the needle shield toward the first axial position and the third axial position. The spring is grounded proximally on the needle hub and is grounded distally on the proximal end of the ribs.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limited of the present invention, and wherein:
- Figure 1: shows an isometric view of an exemplary embodiment of a needle safety device and a medicament delivery device.
- Figure 2: shows an exploded view of an exemplary embodiment of a needle safety device and a medicament delivery device.
- Figure 3: shows a sectional view of an exemplary embodiment of a needle safety device before use.
- Figure 4: shows a sectional view of an exemplary embodiment of a needle safety device during use.
- Figure 5: shows a sectional view of an exemplary embodiment of a needle safety device after use.

Corresponding parts are marked with the same reference symbols in all figures.

### Detailed Description

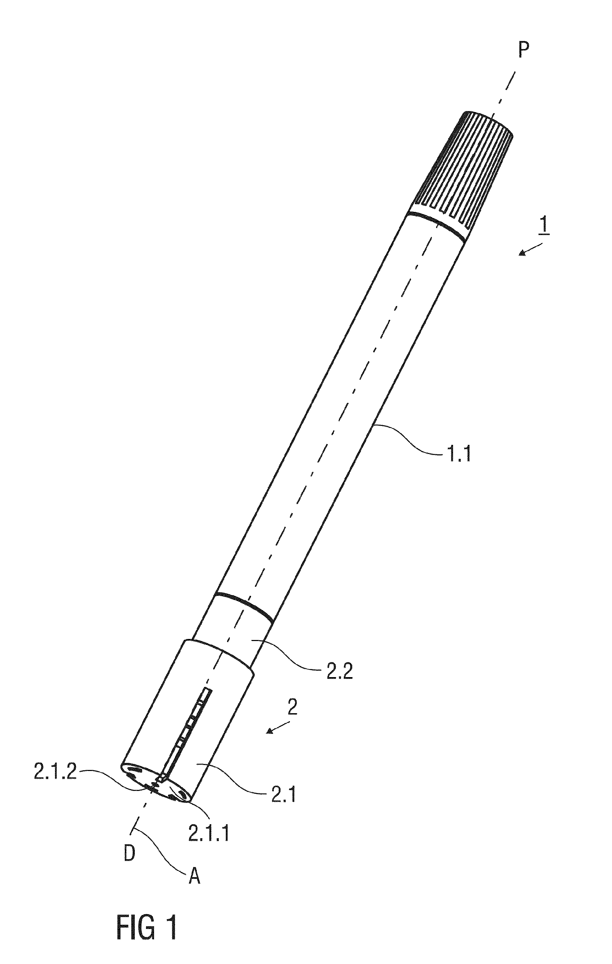

Figure 1 shows an isometric view of an exemplary embodiment of a needle safety device 2 coupled to a medicament delivery device 1. In the exemplary embodiment shown, the delivery device 1 is arranged as a pen injector with an elongated, cylindrical housing 1.1. In other exemplary embodiments, the delivery device 1 may be a syringe, a dental syringe, an auto-injector or a similar device suitable for delivering a medicament.

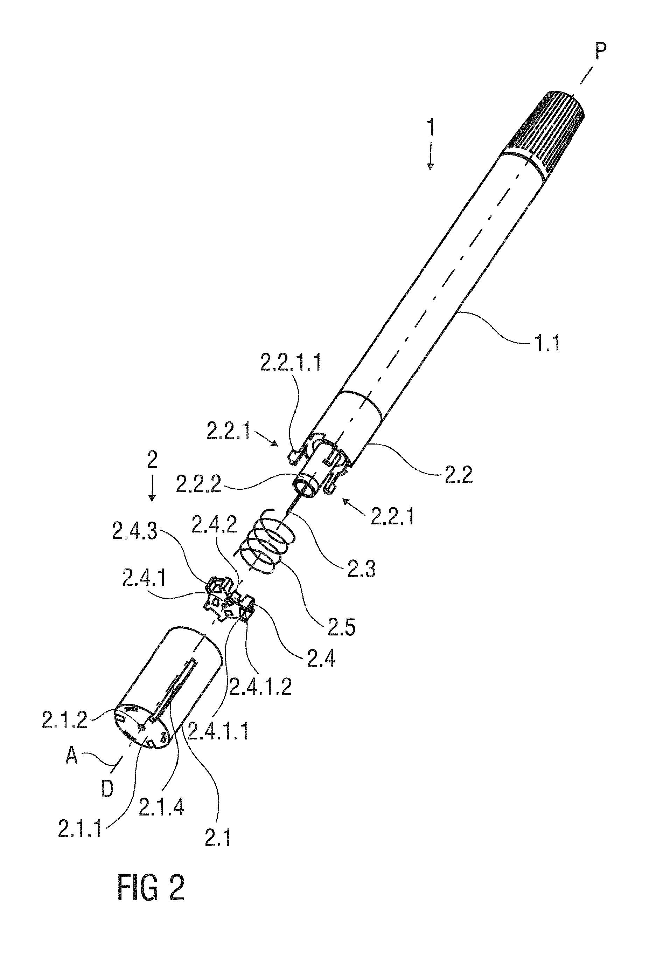

Figure 2 shows an exploded view of an exemplary embodiment of the needle safety device 2. The needle safety device 2 comprises a needle shield 2.1, a needle hub 2.2, a needle 2.3, a locking element 2.4 and a spring 2.5.

In an exemplary embodiment, the needle shield 2.1 includes a distal face 2.1.1 having a first aperture 2.1.2 arranged to allow a distal tip 2.3.1 of the needle 2.3 to pass through during an injection procedure (a proximal tip 2.3.2 of the needle 2.3 is adapted to pierce a septum of a container of medicament in the delivery device 1). One or more slots 2.4 having a distal end 2.1.4.1 and a proximal end 2.1.4.2 may be formed in the needle shield 2.1. An internal surface of the needle shield 2.1 may include one or more axial ribs 2.1.3 having a proximal end 2.1.3.2 and a distal end 2.1.3.1. The distal end 2.1.3.1 may be ramped radially away from the axis A to create an indent on a distal end of the needle shield 2.1. The needle shield 2.1 may be generally cylindrical in shape and move relative to the needle hub 2.2 during an injection procedure.

In an exemplary embodiment, the needle hub 2.2 may be removably coupled to the delivery device 1 (e.g., by a thread 2.2.3, snap-fit, bayonet fit, friction fit, etc.) or integrally formed on a distal end of the delivery device 1. The needle hub 2.2 includes arms 2.2.1 extending distally. Each arm 2.2.1 includes a radial protrusion 2.2.1.1 which is adapted to engage one of the slots 2.4 on the needle shield 2.1. The engagement of the radial protrusions 2.2.1.1 and the slots 2.4 prevent the needle shield 2.1 from separating from the needle hub 2.2. The arms 2.2.1 may extend axially (in an axis parallel to a longitudinal axis A of the needle safety device 2) from a circumference of the needle hub 2.2. The needle hub 2.2 also includes a stem for holding the needle 2.3. The stem may include a recess 2.2.2 arranged as, for example, an annular indent.

In an exemplary embodiment, the locking element 2.4 includes a center portion 2.4.1 with projections 2.4.1.1 extending radially therefrom. Between the projections 2.4.1.1 may be spaces 2.4.1.2. The projections 2.4.1.1 may be arranged on or between the ribs 2.1.3 in the needle shield 2.1. The engagement of the projections 2.4.1.1 and the ribs 2.1.3 may prevent the needle shield 2.1 from rotating relative to the needle hub 2.2. The projections 2.4.1.1 may include a resilient engagement element 2.4.3 which is adapted to engage the indent on the distal recess 2.1.4.1 of the rib 2.1.3. The engagement element 2.4.3 may include a ramped surface 2.4.3.1 to engage the ramped surface of the indent. An opposite side of the ramped surface 2.4.3.1 may include an abutment surface 2.4.3.2 adapted to abut the proximal end 2.1.3.2 of the rib 2.1.3. A second aperture 2.4.4 may be formed in the center portion 2.4.1 which is aligned with the first aperture 2.1.2. Resilient latch arms 2.4.2 may extend proximally from the center portion 2.4.1 and be adapted to engage the recess 2.2.2 on the stem of the needle hub 2.2. The latch arms 2.4.2 may include a finger 2.4.2.1 having an abutment surface 2.4.2.2, wherein the finger 2.4.2.1 engages the recess 2.2.2 and the abutment surface 2.4.2.2 prevents distal movement of the locking element 2.4 relative to the stem once engaged.

The spring 2.5 may be grounded proximally on the needle hub 2.2 and distally on a proximal ends 2.1.3.2 of the ribs 2.1.3.

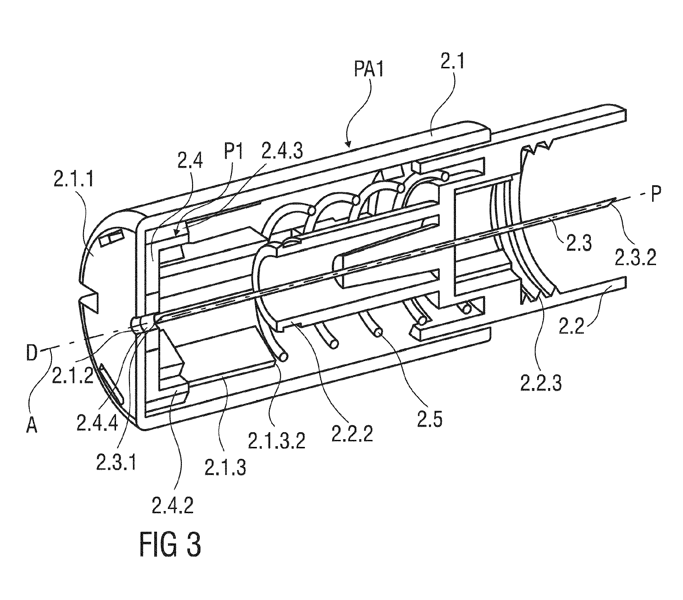

Figure 3 shows an exemplary embodiment of the needle safety device 2 in first axial position (PA1). In the first axial position (PA1), the needle shield 2.1 is extended relative to the needle hub 2.2 and covers the distal tip 2.3.1 of the needle 2.3. The locking element 2.4 is coupled to the needle shield 2.1, because the engagement element 2.4.3 is engaged with the distal end 2.1.3.1 of the ribs 2.1.3. In the first axial position (PA1), the spring 2.5 may not be under any load.

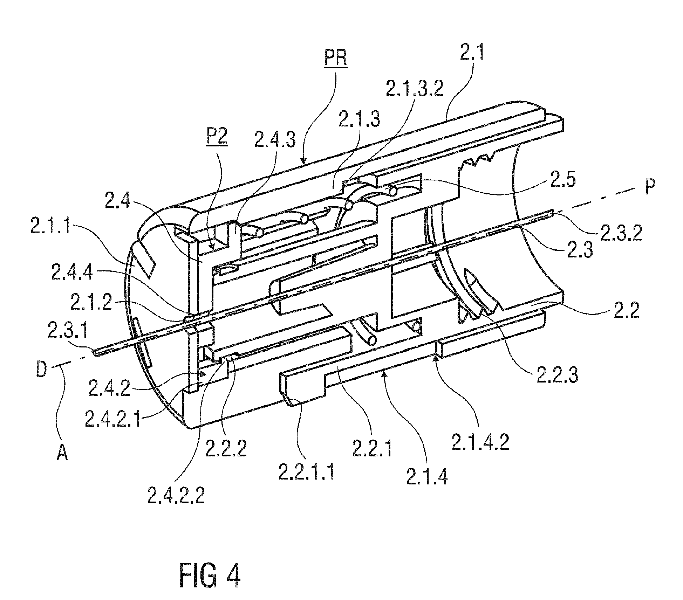

Figure 4 shows an exemplary embodiment of the needle safety device 2 in a second axial position (PA2). In the second axial position (PA2), the needle shield 2.1 is retracted relative to the needle hub 2.2 and the spring 2.5 is compressed, thereby exposing the distal tip 2.3.1 of the needle 2.3 through the first aperture 2.1.2 and the second aperture 2.4.4. When the needle safety device 2 is pressed against an injection site, the proximally directed force causes the engagement element 2.4.3 to deflect radially and disengage the distal end 2.1.3.1 of the rib 2.1.3. The ramped engagement between the engagement element 2.4.3 and the distal end 2.1.3.1 may provide some resistance when the needle safety device 2 is first pressed against the injection site. In the second axial position (PA2), the locking element 2.4 abuts the stem, and the latch arms 2.4.2 engage the recess 2.2.2. That is, the fingers 2.4.2.1 may deflect when the latch arms 2.4.2 abut the stem and remain deflected until the fingers 2.4.2.1 reach the recess 2.2.2 (and then return to the non-deflected position).

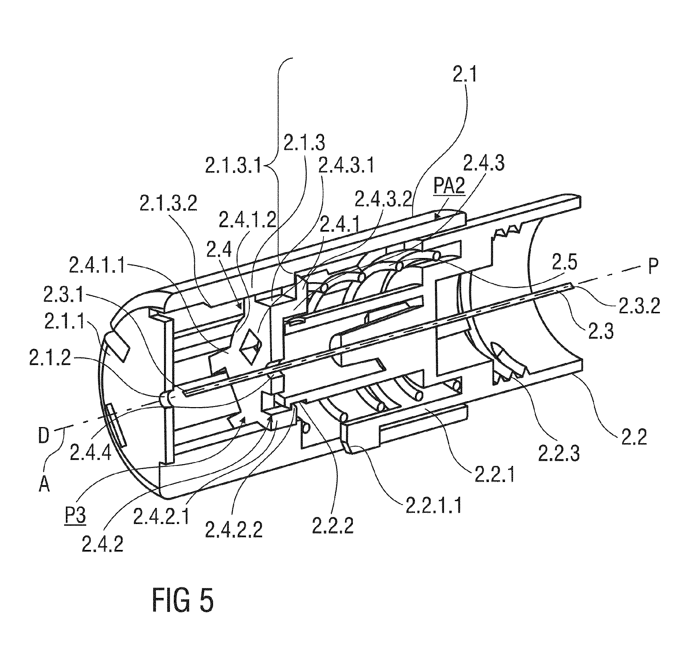

Figure 5 shows an exemplary embodiment of the needle safety device 2 in a third axial position (PA3). In the third axial position (PA3), the needle shield 2.1 is extended relative to the needle hub 2.2 and covers the distal tip 2.3.1 of the needle 2.3. Because the latch arms 2.4.2 of the locking element 2.4 engage the recess 2.2.2 on the stem of the needle hub 2.2 in the second axial position (PA2), when the spring 2.5 expands, the locking element 2.4 remains coupled to the stem and the engagement elements 2.4.3 remain deflected by the ribs 2.1.3. When the needle shield 2.1 has moved distally relative to the needle hub 2.2 such that the proximal end 2.1.3.2 of the ribs 2.1.3 bypass the engagement elements 2.4.3, the engagement elements 2.4.3 return to their non-deflected position. The proximal end 2.1.3.2 of the ribs 2.1.3 will abut the engagement elements 2.4.3 if the needle shield 2.1 is moved proximally relative to the needle hub 2.2 after the needle safety device 2 is in the third axial position. Thus, in the third axial position, the needle safety device 2 is needle-safe, because the distal tip 2.3.1 will remain covered by the needle shield 2.1.

A removable film may be disposed on the distal face 2.1.1 of the needle shield 2.1, e.g., to maintain sterility of the needle 2.3.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (2) comprising:
a needle hub (2.2);
a needle (2.3) coupled to the needle hub (2.2), the needle (2.3) having a distal tip (2.3.1);
a needle shield (2.1) telescopically coupled to the needle hub (2.2), the needle shield (2.1); and
a locking element (2.4) including latch arms (2.4.2) adapted to engage the needle hub (2.2) and engagement elements (2.4.3) adapted to engage the needle shield (2.1),
wherein, when the needle shield (2.1) is in a first axial position (PA1) relative to the needle hub (2.2), the needle shield (2.1) covers the distal tip (2.3.1) of the needle (2.3) and the engagement elements (2.4.3) of the locking element (2.4) engage the needle shield (2.1),
wherein, when the needle shield (2.1) is in a second axial position (PA2) relative to the needle hub (2.2), the needle shield (2.1) is retracted to expose the distal tip (2.3.1) of the needle (2.3) and the latch arms (2.4.2) engage the needle hub (2.2), and
wherein, when the needle shield (2.1) is in a third axial position (PA3) relative to the needle hub (2.2), the needle shield (2.1) covers the distal tip (2.3.1) of the needle (2.3) and the engagement elements (2.4.3) abut the needle shield (2.1) to prevent proximal movement of the needle shield (2.1) relative to the needle hub (2.2).

2. The needle safety device (2) according to claim 1, wherein the needle hub (2.2) includes a stem having a recess (2.2.2) adapted to receive the latch arms (2.4.2) of the locking element (2.4).

3. The needle safety device (2) according to claim 2, wherein, when the needle shield (2.1) is moved from the first axial position (PA1) to the second axial position (PA2), the latch arms (2.4.2) are deflected by the stem.

4. The needle safety device (2) according to claim 3, wherein, when the needle shield (2.1) reaches the second axial position (PA3), the latch arms (2.4.2) return to a non-deflected position to engage the recess (2.2.2).

5. The needle safety device (2) according to claims 1, 2 or 3, wherein the latch arms (2.4.2) include fingers (2.4.2.1) adapted to engage the recess (2.2.2).

6. The needle safety device (2) according to any one of the preceding claims, wherein the needle hub (2.2) includes axial arms (2.2.1) having radial protrusions (2.2.1.1) adapted to engage axial slots (2.1.4) on the needle shield (2.1).

7. The needle safety device (2) according to any one of the preceding claims, wherein the needle shield (2.1) includes axial ribs (2.1.3) formed an inner surface.

8. The needle safety device (2) according to claim 7, wherein the ribs (2.1.3) include a distal end (2.1.3.1) adapted to engage the engagement elements (2.4.3) in the first axial position (PA1) and a proximal end (2.1.3.2) adapted to abut the engagement elements (2.1.3) in the third axial position (PA3).

9. The needle safety device (2) according to claim 8, wherein the distal end (2.1.3.1) includes a ramped surface adapted to engage a corresponding ramped surface (2.4.3.1) of the engagement elements (2.4.3).

10. The needle safety device (2) according to claims 7, 8 or 9, wherein, when the needle shield (2.1) is moved from the first axial position (PA1) to the second axial position (PA2), the engagement elements (2.4.3) are deflected by the ribs (2.1.3).

11. The needle safety device (2) according to claim 8 and 10, wherein, when the needle shield (2.1) reaches the third axial position (PA3), the engagement elements (2.4.3) return to a non-deflected position to engage the proximal end (2.1.3.2) of the ribs (2.1.3).

12. The needle safety device (2) according to any one of the preceding claims, further comprising:
a spring (2.5) disposed between the needle hub (2.2) and the needle shield (2.1), the spring (2.5) biasing the needle shield (2.1) toward the first axial position (PA1) and the third axial position (PA3).

13. The needle safety device (2) according to claim 8 and 12, wherein the spring (2.5) is grounded proximally on the needle hub (2.2) and is grounded distally on the proximal end (2.1.3.2) of the ribs (2.1.3).
